# EUROPEAN PATENT SPECIFICATION

(11) **EP 0 460 069 B1**
(45) Date of publication and mention of the grant of the patent: **17.01.1996**
(21) Application number: 90904129.5
(22) Date of filing: 22.02.1990
(51) Int. Cl.: A61B 17/00

(54) **ACOUSTIC IMPACT DELIVERY CATHETER WITH END CAP**
AKUSTISCHE STOSSWELLEN AUSSENDENDER KATHETER MIT ENDKAPPE
CATHETER D'ACHEMINEMENT D'IMPACT ACOUSTIQUE A OBTURATION D'EXTREMITE

(30) Priority: 22.02.1989 US 314472
(43) Date of publication of application: 11.12.1991
(73) Proprietor: CONNECTICUT INNOVATIONS, INC., Rocky Hill, Connecticut 06067 (US); THE GENERAL HOSPITAL CORPORATION, Boston MA 02114 (US)
(72) Inventor: ROSEN, David, I., Peabody, MA 01960 (US); PETSCHEK, Harry, Lexington, MA 02173 (US); DRETLER, Stephen, P., Wayland, MA 01778 (US); BHATTA, Krishna, M., Brookline, MA 02146 (US)
(74) Representative: Modiano, Guido, Dr.-Ing.
(86) International application number: US9000904
(87) International publication number: WO9009762

(56) References cited:
- EP-A- 0 268 019
- DE-A- 847 950
- FR-A- 2 412 301
- US-A- 3 785 382
- US-A- 3 823 717
- US-A- 4 191 189
- US-A- 4 196 736
- US-A- 4 605 003
- US-A- 4 722 340
- US-A- 4 870 953
- "Mechanism of Laser-Induced Fragmentation of Urinary and Biliary Calculi", (NISHIOKA et al) 1987 (Note Figures 1, 2, pages 233-234).

## Description

### FIELD OF THE INVENTION

The present invention relates to an apparatus for fracturing hard formations in the body, and more specifically, to an apparatus for transferring energy to the end cap of a flexible guide which imparts a high-velocity impulse to a target deposit thereby fracturing it.

### BACKGROUND OF THE INVENTION

Calciferous and similar deposits occur in body fluid passages of various types. Of particular interest are kidney and gall stones as well as arterial plaque.

Radiation in various forms has been used for destroying or removing such deposits from the internal passages of the body. In one form of laser therapy, radiation is directed onto a light-receiving surface of a heat-generating element. The element is then placed in contact with the target deposit, melting it. This approach has several drawbacks which include:
1. thermal damage to surrounding tissue;
2. only fatty plaques readily melt;
3. more advanced fibrous and calcified plaques form char and debris; and
4. the hot element adheres to the tissue rupturing it when the element is removed.

In another approach, laser radiation is applied directly to the target deposit to ablate it or produce shock waves that induce fragmentation. Direct lasertripsy has several disadvantages. Laser energy often damages healthy tissue surrounding the target deposit by direct absorption or by acting as a general heat sink for the high temperature plasma. Some deposits only weakly absorb radiation thereby requiring greater radiation exposure and damage. A sharp laser delivery fiber can cause damage if inadvertently jabbed into healthy tissue.

US-A-4 605 003 discloses a lithotriptor device used for crushing a stone in an organ of a living body. The device includes a piston, for crushing engagement with the stone, actuated by the combustion of an explosive layer inside the piston cylinder. The explosive layer is exhausted in one firing, and the device may be fired only once before being removed from the body.

US-A-3 785 382 discloses a device for destroying bladder and kidney stones etc. which produces hydraulic shock waves in a fluid reservoir located in a housing which remains outside the body. These shock waves are transmitted along a lithotriptor which extends into the body.

The present invention contemplates an apparatus as defined in either claim 1 or 10 for selectively fracturing hard deposits in fluid contained in body passages with the impact of small jack hammer like blows from a capped flexible guide inserted through the body passage to the location of a deposit to be fractured.

In implementing the invention, a flexible wire guide terminating with a hard mass or end cap is provided for insertion through a fluid containing body passage. An energy source and delivery system in the flexible guide provide a pulse of energy in the vicinity of the cap to produce a rapid vapor expansion that causes the end cap to undergo a pulse like movement as the vapor expands against the fluid medium of the passage to impart a high-velocity impulse to the target deposit. A means for fluid exchange between the interior and exterior of the apparatus is provided in the end cap region to insure the presence of fluid for increasing the direct impulse against the cap.

In a first embodiment of the invention, the energy source is a pulsed laser and the delivery system is an optical fiber passing through the guide to terminate adjacent to a metal end cap. The laser energy causes vaporization of a small portion of the end cap to create the vapor expansion that drives the end cap forward against the inertia of the fluid.

In a second embodiment, the energy source is a pulsed voltage source and a pair of conductors, one of which may be the wire guide, comprising the delivery system. The conductors terminate in a spark gap adjacent to the end cap. A spark pulse causes fluid vaporization adjacent to the end cap, thereby driving it forward as a reaction.

An advantage of the invention includes the end cap's protection of surrounding healthy tissue from direct laser radiation and thermal radiation from the laser-produced plasma of the vapor expansion which forms against the inside surface of the end cap. A further advantage of the invention is the elimination of inadvertent puncturing of healthy tissue by a sharply pointed laser delivery fiber.

In the laser embodiment, the end cap is fabricated to exhibit good laser absorption providing a reliable, reproducible vapor expansion independent of the absorption characteristics of the target deposit.

### DESCRIPTION OF THE DRAWINGS

The invention will be more fully understood from the following solely exemplary detailed description taken in conjunction with the accompanying drawing, in which;
Fig. 1 is a diagram of one embodiment of the invention which utilizes a laser-produced vapor expansion to drive a hard mass to contact and fragment a target deposit;
Fig. 2 is a diagram of a second embodiment of the invention which utilizes an electrically-produced spark discharge that produces a vapor expansion to drive a hard mass, to contact and fragment a target deposit;
Fig. 3 is a diagram of a variant of the invention of Fig. 2 which utilizes an electrically-produced plasma from a central two wire conductor to drive an internally confined spring-loaded end cap to fragment a target deposit;
Fig. 4 is a diagram of a further variant of the invention of Fig. 2 which utilizes an electrically-produced plasma from a central two wire conductor to drive an internally confined stainless steel end cap with irrigation ports, to contact and fragment a target deposit;
Fig. 5 is a diagram of the fragmentation system of the invention inside a body passage with fragmented target deposits at its distal end and a positioning fluoroscope; and
Fig. 6 is a diagram of an end cap of the invention demonstrating the pulse-like advancement of the end cap with subsequent fracturing of the target deposit.

### DETAILED DESCRIPTION OF THE INVENTION

The present invention contemplates a method and apparatus for driving a small, hard mass into fracturing, high velocity contact with hard deposits in fluid-containing body passages.

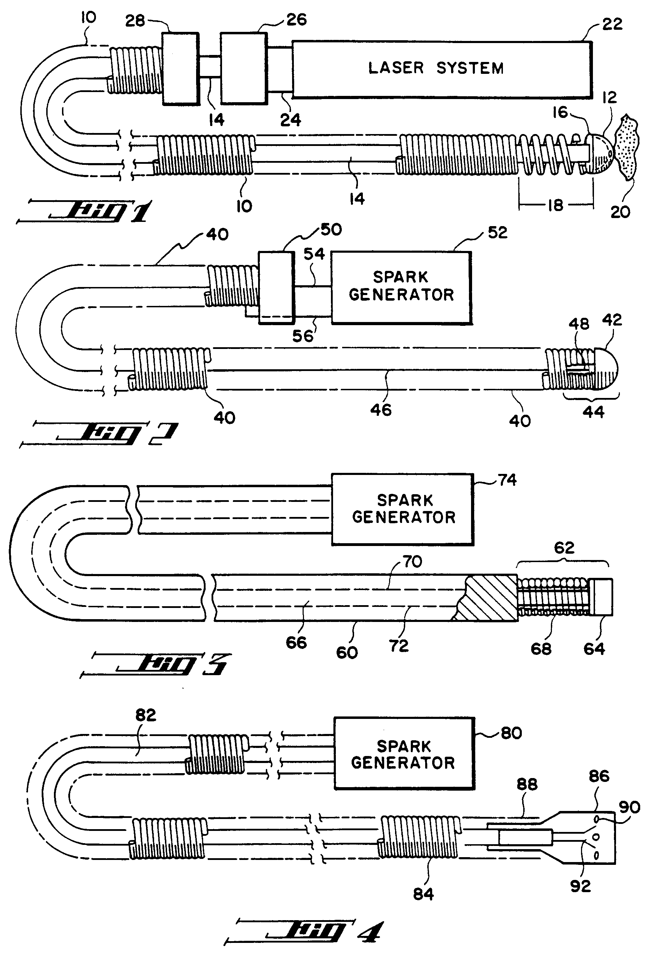

A first embodiment of the invention is illustrated in Fig. 1. A flexible wire guide 10, which typically comprises a commercially available, helically wound French No. 3 guide (0.85 - 1.00m diameter) has an end cap 12, the cap and guide are typically of stainless steel or other material which, in response to laser radiation, will vaporize, in a rapid vapor expansion. An optical fiber 14 (typically a 200 micron core) is fed through the wire guide 10 and terminates at a point 16, a short distance from the end cap 12. Laser radiation emanating from the termination 16 strikes the end cap and is absorbed by the metal of the end cap 12, causing vaporization of a small portion of the metal. In actual use, the helical windings of the guide 10 are opened in a terminal portion 18, for example by stretching the last few coils, to facilitate the entry of fluid from body passages, in which the guide is inserted, into the region of the termination 16. The rapid vapor expansion, typically of shock wave nature, generates a forward pressure impulse on the cap 12, in the nature of a miniature jack hammer.

Where the cap 12 has been inserted in a body passage, typically the urethra to a kidney stone, it is capable of fragmenting pieces of the kidney stone, such as the stone 20, to which it comes into contact. The presence of the body fluid creates a mass within the region of the termination 16 where the vapor expansion occurs which confines the expansion and permits a large portion of the energy of the vapor expansion to be directed against the end cap 12 producing a high-velocity, short forward impulse.

The source of radiation applied to the fiber 14 is a laser system 22. Laser system 22 is typically a tunable dye laser. The laser is operated in the mode of producing pulses of approximately 1 microsecond duration and approximately 50 millijoule energy level. Other pulsed laser systems capable of promptly initiating a plasma against the cap and compatible with optical fiber transmission would also be acceptable energy sources. This would include, for example, solid state laser systems such as Alexandrite. A pulsed output beam 24 from the laser 22 is applied to an optical coupling system 26 which in turn applies the pulsed radiation in beam 24 onto optical fiber 14. The fiber 14 passes through a clamp 28 connected to the guide 10 and operative to hold the fiber termination 16 at a predetermined distance from the cap 12. A potting compound near the termination may be used to secure the fiber termination.

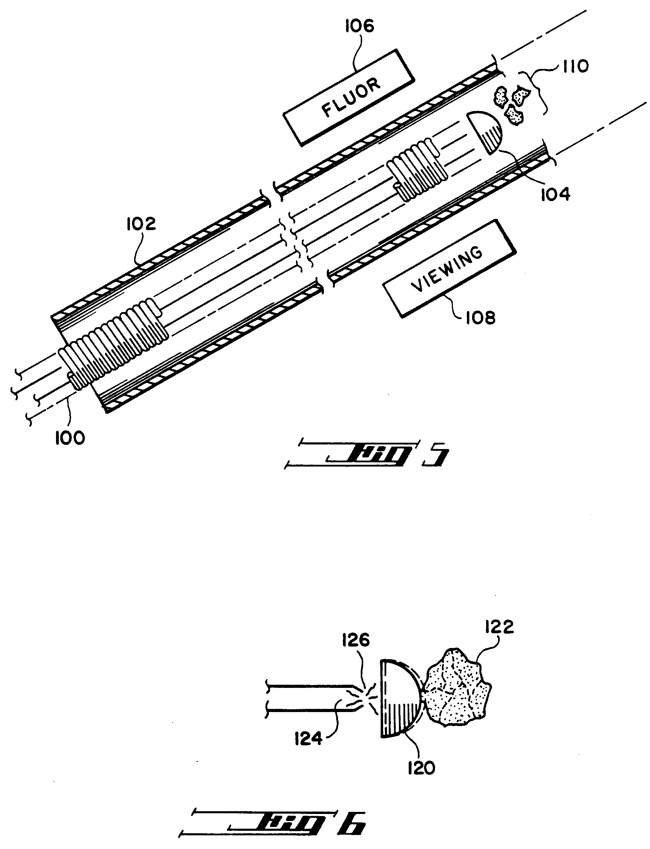

A second embodiment of the invention is illustrated in Fig. 2. As shown there a wire guide 40, which may be similar to the guide 10 in Fig. 1 and typically of a size corresponding to French No. 3, terminates in an end cap 42 at a distal end 44 of the wire guide 40. The wire guide is typically helically wound as described before and the termination 44 has helical wires of augmented spacing, for example, by being stretched, to permit the flow of fluid through an interior portion.

A wire conductor 46 is inserted through the wire guide 40, spaced and insulated from the helically wound wires of the guide 40. The inner conductor 46 terminates at a point 48, adjacent to the cap 42. The wire may be held in place by a positioning clamp 50, or potted in place with an adhesive as described above with respect to Fig. 1.

A spark generator 52, which can be a Wolfe 2137.50 or Northgate Research SDl, available in the art, has its output applied on conductors 54 and 56, the conductors are connected to the inner conductor 46 of the guide 40 and the outer helical windings of the guide 40. The spark generator 52 produces an output pulse of up to several microseconds, at several KV and up to lKA current. The spark generated between the termination 48 of the inner conductor 46 and the end cap 42 causes a vapor expansion of the fluid entering the tip portion 44 and/or the metal of the cap 42 creating a jack hammer like shock impulse movement of the end cap 42, permitting it to fracture calciferous deposits which it contacts.

A different version of the embodiment illustrated in Fig. 2 is shown with respect to Fig. 3. A wire guide 60, typically of the type illustrated above, though not necessarily having a conducting outer shell, has a termination 62 which may be an open helical portion of the wire guide of the prior embodiments. An end cap 64 is applied to the distal end of the wire guide 60. A dual conductor transmission line 66 passes centrally through the wire guide 60 terminating at a point 68 adjacent to the cap 64. The transmission line 66 contains first and second conductors 70 and 72 which terminate to provide a spark gap at the termination 68. The gap is selected to provide, in response to energization from a spark generator 74, of the type illustrated above with respect to Fig. 2, a vaporization of the fluid within the terminal portion 62 generating an impulse motion of the cap 64. A transmission line of the type provided with the above-identified supplier of the spark generator and intended for independent insertion into body passages is suitable for insertion within the guide 60.

Fig. 4 illustrates a further version of the embodiment of Fig. 2. A spark generator 80 is provided, and a two conductor transmission line 82 conducts the output of the spark generator through a guide 84 into a stainless steel end cap 86. The end cap 86 is typically cemented to the distal end 88 of the wire guide 84. Apertures 90 are provided in the end cap 86 to permit body passage fluids to enter the interior of the end cap 86 to a point 92 where the conductors in the transmission cable 92 terminate in a spark gap. The terminal portions of the end cap 86 wire guide 84, where it connects to the end cap 86, are typically resilient enough to permit the impulses generated by the spark from the spark gap 92 termination to drive the end cap 86 forward in jack hammer fashion to permit fracturing of hard deposits to which it is directed.

In actual use, and as illustrated in Fig. 5, a wire guide 100 according to the present invention is inserted through a body passage 102 such as the urethra, for kidney stone fracturing, the biliary duct for gall stone fracturing and an artery for arterial plaque break-up. The end tip 104 of the wire guide 100 is guided by fluoroscopy. An X-ray source 106 and viewing display 108 permit the end of the wire guide 100 to be positioned adjacent to a hard deposit 110 to be fractured as illustrated.

Fig. 6 is an illustration of the dynamics by which an end cap 120 is jack hammered or shock driven forward into a hard deposit 122 by a spark generated by a discharge in a spark gap 126.

Impulse delivery systems other than laser or spark, such as chemical reaction, trigger remotely by signals supplied by a guide, may be used.

It will be understood that all matter herein described or shown in the accompanying drawings is to be interpreted as illustrative only and is not to limit the invention defined in the following claims.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. An apparatus for fracturing hard formations in the body comprising:
a guide (10; 40; 60) adapted for insertion through a body passage (102) containing a body fluid, said guide having a terminal portion (18; 44; 62) defining an interior space, said terminal portion being located within the body passage when said guide is in an operative position;
an end cap (12; 42; 64) movably coupled to said terminal portion so that the end cap may move between a first position located adjacent to said terminal portion and a second position separated from said first position; and
a source of energy (22; 52; 74) for producing an energy pulse, characterized in that
said guide is a flexible guide;
said terminal portion of said guide is adapted for receiving body fluid from said body passage; and in that
the energy of said pulse generated by the source of energy produces a rapid vaporization of said body fluid located within the terminal portion to move said end cap from the first position into the second position, whereby said end cap performs a series of movements between said first and second positions.

2. Apparatus as set forth in claim 1 wherein said source of energy (22; 52; 74) is a spark generator.

3. Apparatus as set forth in claim 2 wherein said spark generator (52; 74) includes a first electrical conductor (46; 70) extending through said flexible guide (10; 40; 60) for applying sparks to the terminal portion (18; 44; 62) .

4. Apparatus as set forth in claim 3 further comprising a second electrical conductor (72) which extends through said flexible guide (10; 40; 60), wherein a spark gap (126) is located at an end of said first and second electrical conductors.

5. Apparatus as set forth in claim 1 wherein said source of energy is a laser (22) adapted for producing a series of pulses of radiant energy, each pulse of radiant energy producing said rapid vaporization of said fluid in said terminal portion (18) to move said end cap (12) from said first position into said second position, so that the series of pulses of radiant energy cause a corresponding series of movements of the end cap between said first and second positions.

6. Apparatus as set forth in claim 5 further comprising an optical fiber (14) extending through the flexible guide (10) for transmitting the pulses of radiant energy from the laser (12) to the terminal portion (18).

7. Apparatus as set forth in one or more of the preceding claims wherein said flexible guide (10; 40; 60) has an outside diameter of 0.85 to 1.00 mm.

8. Apparatus as set forth in one or more of the preceding claims wherein said terminal portion (18; 44; 62) includes an expanded end of said flexible guide (10; 40; 60) for providing fluid access to an interior of said terminal portion.

9. Apparatus as set forth in one or more of the preceding claims further including means for returning said end cap (12; 42; 64) to said first position after said end cap has been moved into said second position.

10. An apparatus for fracturing hard formations in the body comprising:
a guide (84) adapted for insertion through a body passage (102) containing a body fluid, said guide having a terminal portion (88) defining an interior space, said terminal portion being located within the body passage when said guide is in an operative position;
an end cap (86) movably coupled to said terminal portion so that the end cap may move between a first position located adjacent to said terminal portion and a second position separated from said first position; and
a source of energy (80) for producing an energy pulse, characterized in that
said guide is a flexible guide;
said end cap includes apertures (90) for permitting body passage fluids entering said end cap; and in that
the energy of said pulse generated by the source of energy produces a rapid vaporization of said body fluid located within said end cap to move said end cap from the first position into the second position, whereby said end cap performs a series of movements between said first and second positions.

11. Apparatus as set forth in claim 10 wherein said source of energy (80) includes a spark generator and wherein a two conductor transmission line (82) conducts the output of the spark generator into said end cap (86).

12. Apparatus as set forth in claim 11 wherein line (82) terminates in a spark gap placed in the interior of said end cap (86).

## Patentansprüche

1. Ein Gerät zum Zertrümmern von festen Ablagerungen im Körper, das umfaßt:
eine Führung (10; 40; 60), die zum Einsetzen durch eine Körperöffnung (102) ausgebildet ist, die eine Körperflüssigkeit enthält, wobei die Führung einen Endabschnitt (18; 44; 62) aufweist, der einen Innenraum definiert, wobei sich der Endabschnitt innerhalb der Körperöffnung befindet, wenn sich die Führung in einer Betriebsstellung befindet,
eine Endkappe (12; 42; 64), die verschiebbar am Endabschnitt befestigt ist, so daß sich die Endkappe zwischen einer ersten Stellung, die an den Endabschnitt angrenzt, und einer zweiten Stellung, die von der ersten Stellung getrennt ist, bewegen kann; und
eine Energiequelle (22; 52; 74) zur Erzeugung eines Energieimpulses, dadurch gekennzeichnet, daß
die Führung eine elastische Führung ist, wobei der Endabschnitt der Führung dafür ausgebildet ist, um Körperflüssigkeit aus der Körperöffnung aufzunehmen; und dadurch, daß
die Energie des Impulses, das durch die Energiequelle erzeugt wird, eine schnelle Verdampfung der Körperflüssigkeit bewirkt, die sich innerhalb des Endabschnitts befindet, um die Endkappe von der ersten Stellung in die zweite Stellung zu bewegen, wodurch die Endkappe eine Reihe von Bewegungen zwischen der ersten und der zweiten Stellung durchführt.

2. Das Gerät gemäß Anspruch 1, worin die Energiequelle (22; 52; 74) ein Funkengenerator ist.

3. Das Gerät gemäß Anspruch 2, worin der Funkengenerator (52; 74) einen ersten elektrischen Leiter (46; 70) umfaßt, der sich durch die elastische Führung (10; 40; 60) erstreckt, um Funken an den Endabschnitt (18; 44; 62) anzulegen.

4. Das Gerät gemäß Anspruch 3, das weiterhin einen zweiten elektrischen Leiter (72) umfaßt, der sich durch die elastische Führung (10; 40; 60) erstreckt, worin sich eine Funkenstrecke (126) an einem Ende des ersten und zweiten elektrischen Leiters befindet.

5. Das Gerät gemäß Anspruch 1, worin die Energiequelle ein Laser (22) ist, der geeignet ist, um eine Reihe von Strahlungsenergieimpulsen zu erzeugen, wobei jeder Strahlungsenergieimpuls die schnelle Verdampfung der Flüssigkeit im Endabschnitt (18) erzeugt, um die Endkappe (12) von der ersten Stellung in die zweite Stellung zu bewegen, so daß die Reihe von Strahlungsenergieimpulsen eine entsprechende Reihe von Bewegungen der Endkappe zwischen der ersten und zweiten Stellung bewirkt.

6. Das Gerät gemäß Anspruch 5, das weiterhin eine Lichtleitfaser (14) umfaßt, die sich durch die elastische Führung (10) erstreckt, um die Strahlungsenergieimpulse vom Laser (12) zum Endabschnitt (18) zu übertragen.

7. Das Gerät gemäß einem oder mehreren der vorangegangenen Ansprüche, worin die elastische Führung (10; 40; 60) einen Außendurchmesser von 0,85 bis 1,00 mm aufweist.

8. Das Gerät gemäß einem oder mehreren der vorangegangenen Ansprüche, worin der Endabschnitt (18; 44; 62) ein ausgeweitetes Ende der elastischen Führung (10; 40; 60) umfaßt, um einen Flüssigkeitszufluß zum Inneren des Endabschnitts bereitzustellen.

9. Das Gerät gemäß einem oder mehreren der vorangegangenen Ansprüche, das weiterhin Mittel zur Rückführung der Endkappe (12; 42; 64) in die erste Stellung umfaßt, nachdem die Endkappe in die zweite Stellung bewegt wurde.

10. Ein Gerät zum Zertrümmern fester Ablagerungen im Körper, das umfaßt:
eine Führung (84), die zum Einsetzen durch eine Körperöffnung (102) ausgebildet ist, die eine Körperflüssigkeit enthält, wobei die Führung einen Endabschnitt (88) aufweist, der einen Innenraum definiert, wobei sich der Endabschnitt innerhalb der Körperöffnung befindet, wenn die Führung in einer Betriebsstellung ist;
eine Endkappe (86), die verschiebbar mit dem Endabschnitt gekoppelt ist, so daß sich die Endkappe zwischen einer ersten Stellung, die an den Endabschnitt angrenzt, und einer zweiten Stellung, die von der ersten Stellung getrennt ist, bewegen kann; und
eine Energiequelle (80) zur Erzeugung eines Energieimpulses, dadurch gekennzeichnet, daß
die Führung eine elastische Führung ist, wobei die Endkappe Öffnungen (90) umfaßt, um zu erlauben, daß die durch die Körperöffnung fließenden Flüssigkeiten in die Endkappe eindringen; und dadurch, daß
die Energie des durch die Energiequelle erzeugten Impulses eine schnelle Verdampfung der Körperflüssigkeit bewirkt, die sich innerhalb der Endkappe befindet, um die Endkappe von der ersten Stellung in die zweite Stellung zu bewegen, wodurch die Endkappe eine Reihe von Bewegungen zwischen der ersten und der zweiten Stellung durchführt.

11. Das Gerät gemäß Anspruch 10, worin die Energiequelle (80) einen Funkengenerator umfaßt, und worin eine zweiadrige Übertragungsleitung (82) die Ausgabe des Funkengenerators in die Endkappe (86) leitet.

12. Das Gerät gemäß Anspruch 11, worin die Leitung (82) in einer Funkenstrecke endet, die im Inneren der Endkappe (86) angelegt ist.

## Revendications

1. Appareil pour fracturer des formations dures dans le corps, comprenant :
un guide (10 ; 40 ; 60) adapté pour être inséré dans un passage (102) du corps contenant un fluide corporel, ledit guide présentant une partie terminale (18 ; 44 ; 62) formant un volume intérieur, ladite partie terminale étant située dans le passage du corps quand ledit guide est en position de travail ;
un embout d'extrémité (12 ; 42 ; 64) accouplé de façon mobile à ladite partie terminale pour que l'embout d'extrémité puisse se déplacer entre une première position située au voisinage immédiat de ladite partie terminale et une seconde position espacée à ladite première position ; et
une source d'énergie (22 ; 52 ; 74) pour produire une impulsion d'énergie, caractérisé en ce que :
ledit guide est un guide flexible ;
ladite partie terminale dudit guide est adaptée pour recevoir un fluide corporel provenant dudit passage du corps ; et en ce que
l'énergie de ladite impulsion engendrée par la source d'énergie produit une vaporisation rapide dudit fluide corporel situé dans la partie terminale pour déplacer ledit embout d'extrémité de la première position à la seconde position, moyennant quoi ledit embout d'extrémité effectue une série de déplacements entre lesdites première et seconde positions.

2. Appareil selon la revendication 1, dans lequel ladite source d'énergie (22 ; 52 ; 74) est un générateur à étincelles.

3. Appareil selon la revendication 2, dans lequel ledit générateur à étincelles (52 ; 74) comporte un premier conducteur électrique (46 ; 70) s'étendant dans ledit guide flexible (10 ; 40 ; 60) pour appliquer des étincelles à la partie terminale (18 ; 44 ; 62).

4. Appareil selon la revendication 3, comprenant en outre un second conducteur (72) qui s'étend dans ledit guide flexible (10 ; 40 ; 60), un intervalle d'étincelage (126) étant situé à une extrémité desdits premier et second conducteurs électriques.

5. Appareil selon la revendication 1, dans lequel ladite source d'énergie est un laser (22) adapté pour produire une série d'impulsions d'énergie de rayonnement, chaque impulsion d'énergie de rayonnement produisant ladite vaporisation rapide dudit fluide dans ladite partie terminale (18) pour déplacer ledit embout d'extrémité (12) de ladite première position à ladite seconde position, de sorte que la série d'impulsions d'énergie de rayonnement entrai'ne une série correspondante de déplacements de l'embout d'extrémité entre lesdites première et seconde positions.

6. Appareil selon la revendication 5, comprenant en outre une fibre optique (14) s'étendant dans le guide flexible (10) pour transmettre les impulsions d'énergie de rayonnement du laser (12) à la partie terminale (18).

7. Appareil selon une ou plusieurs des revendications précédentes, dans lequel ledit guide flexible (10 ; 40; 60) a un diamètre extérieur de 0,85 à 1,00 mm.

8. Appareil selon une ou plusieurs des revendications précédentes, dans lequel ladite partie terminale (18 ; 44 ; 62) comporte une extrémité élargie dudit guide flexible (10 ; 40 ; 60) pour former un accès du fluide vers l'intérieur de ladite partie terminale.

9. Appareil selon une ou plusieurs des revendications précédentes, comprenant en outre des moyens pour renvoyer ledit embout d'extrémité (12 ; 42 ; 64) dans la première position après que ledit embout d'extrémité s'est déplacé dans ladite seconde position.

10. Appareil pour fracturer des formations dures dans le corps, comprenant :
un guide (84) adapté pour être inséré dans un passage (102) du corps contenant un fluide corporel, ledit guide présentant une partie terminale (88) formant un volume intérieur, ladite partie terminale étant située dans le passage du corps quand ledit guide est en position de travail ;
un embout d'extrémité (86) accouplé de façon mobile à ladite partie terminale pour que l'embout d'extrémité puisse se déplacer entre une première position située au voisinage immédiat de ladite partie terminale et une seconde position espacée à ladite première position ; et
une source d'énergie (80) pour produire une impulsion d'énergie, caractérisé en ce que :
ledit guide est un guide flexible ;
ledit embout d'extrémité comporte des ouvertures (90) pour permettre à des fluides d'entrer dans ledit embout d'extrémité du passage du corps ; et en ce que
l'énergie de ladite impulsion engendrée par la source d'énergie produit une vaporisation rapide dudit fluide corporel situé dans ledit embout d'extrémité pour déplacer ledit embout d'extrémité de la première position à la seconde position, moyennant quoi ledit embout d'extrémité effectue une série de déplacements entre lesdites première et seconde positions.

11. Appareil selon la revendication 10, dans lequel ladite source d'énergie (80) comporte un générateur à étincelles et dans lequel une ligne de transmission (82) à deux conducteurs conduit la sortie du générateur à étincelles dans ledit embout d'extrémité (86).

12. Appareil selon la revendication 11, dans lequel la ligne (82) se termine en un intervalle d'étincelage disposé à l'intérieur dudit embout d'extrémité (86).
